(19) Europäisches Patentamt
European Patent Office
Office européen des brevets

(11) Publication number: 0 114 908 B1

(12) **EUROPEAN PATENT SPECIFICATION**

(45) Date of publication of patent specification: **21.05.86**

(51) Int. Cl.⁴: **A 61 K 31/53, C 07 D 251/18**

(21) Application number: **83100753.9**

(22) Date of filing: **27.01.83**

(54) **Finely pulverized 2,4-diamino-6-(2,5-dichlorophenyl)-1,3,5-triazine and pharmaceutically acceptable acid addition salts thereof.**

(43) Date of publication of application:
**08.08.84 Bulletin 84/32**

(45) Publication of the grant of the patent:
**21.05.86 Bulletin 86/21**

(84) Designated Contracting States:
**CH DE FR GB LI NL SE**

(56) References cited:
**GB-A-1 441 904**
**US-A-4 103 009**

(73) Proprietor: **NIPPON SHINYAKU COMPANY, LIMITED**
**14, Kisshoin Nishinosho Monguchicho**
**Minami-ku Kyoto-shi Kyoto (JP)**

(72) Inventor: **Enomoto, Hiroshi**
**26-3-7-707 Baba Miba Hashiri**
**Nagaokakyo-shi Kyoto-fu 617 (JP)**
Inventor: **Kawamata, Masanobu**
**17-6 Kitanakaincho Saganisonji Monzen**
**Ukyo-ku, Kyoto 616 (JP)**
Inventor: **Nomura, Akira**
**1-1 Higashiyama-2-chome**
**Hirakata-shi Osaka-fu 573 (JP)**
Inventor: **Aoyagi, Yoshiaki**
**8-7-105 Uchidehama**
**Otsu-shi Shiga-ken 520 (JP)**
Inventor: **Ueda, Fusao**
**272-16 Choshoji, Notogawa-cho**
**Kanzaki-gun Shiga-ken 521-12 (JP)**

(74) Representative: **Wey, Hans-Heinrich, Dipl.-Ing. et al**
**Patentanwälte Müller-Börner & Wey**
**Widenmayerstrasse 49**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 114 908 B1

**Description**

2,4-diamino-6-(2,5-dichlorophenyl)-1,3,5-triazine and various acid addition salts thereof are known to exhibit strong anti-ulcer activity (see Japanese Patent Nos. 919103 and 1017236). Benzoguanamine derivatives having the general formula

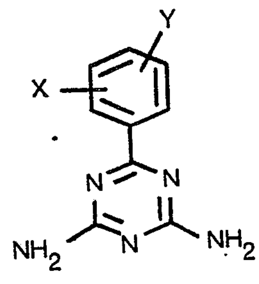

wherein X and Y are halogen atoms other than 3,4-dichloro, are also known from GB—A—1 441 904. These compounds have a low toxicity and valuable pharmacological activities, especially a high anti-peptic ulcer activity. However, in testing these compounds for their anti-ulcer activity, it has been found that disadvantages arise due to lack of reproducibility of the level of anti-ulcer activity when the compounds are administered without regard to the average particle size. In particular, it has been found that the compounds lack a dose dependency thereby giving rise to unpredictability as well as lack of reproducibility of the activity levels.

The present invention is based on the surprising discovery that a number of advantages accrue from finely pulverizing 2,4-diamino-6-(2,5-dichlorophenyl)-1,3,5-triazine or a pharmaceutically acceptable acid addition salt thereof, so that the average particle size is 20 µm (microns) or less. A particularly useful average particle size is 5 to 10 µm (microns), and extremely good results have been achieved wherein the average particle size is about 8 µm (microns).

When 2,4-diamino-6-(2,5-dichlorophenyl)-1,3,5-triazine or a pharmaceutically acceptable acid addition salt thereof is finely pulverized so that the average particle size is 20 µm (microns) or less, a number of quite unexpected advantages result. These advantages include a marked improvement in the properties and reducibility of the activity. For example, the inibition activity against stress ulcer in rats showed linear dose dependency. In addition, quite surprisingly, investigations on the effect of toxicity revealed that the finely pulverized particles according to the present invention were far less toxic than those of the prior art having an average particle size of about 50 µm (microns). In addition, we have observed that body weight increase inhibition which is a main side effect of administration of the compound of the present invention or a pharmaceutically acceptable acid addition salt thereof, is only transient and moderate and the recovery is far faster than occurs when the prior art having an average particle size of about 50 µm (microns) is administered.

The present invention, therefore, resides in the discovery of unexpected improvement in properties and decrease in side effects when 2,4-diamino-6-(2,5-dichlorophenyl)-1,3,5-triazine and pharmaceutically acceptable acid addition salts thereof are finely pulverized so that the average particle size is 20 µm (microns) or less. Also included within the present invention are pharmaceutical compositions useful for treating peptic ulcers in humans and animals which comprises 2,4-diamino-6-(2,5-dichlorophenyl)-1,3,5-triazine or a pharmaceutically acceptable acid addition salt thereof in finely pulverized form so that the average particle size is 20 µm (microns) or less, preferably 5—10 µm (microns), in combination with a pharmaceutically acceptable carrier. According to a further embodiment of the present invention, the compound may be used in the form of its maleate salt. Both the compound and pharmaceutically acceptable acid addition salts thereof have been found to be particularly useful when the average particle size is about 8 µm (microns). The compound may be administered as such or in the form of a pharmaceutically acceptable acid addition salt. The maleate salt has been found to be particularly useful. The compounds and the pharmaceutically acceptable acid addition salts thereof, having an average particle size of about 8 µm (microns), have been shown to be useful according to the present invention.

The advantages of the present invention are demonstrated when 2,4-diamino-6-(2,5-dichlorophenyl)-1,3,5-triazine is administered orally to rats and its activity on ulcer formation induced by stress is measured by the immobilization-water immersion method. According to this test, when the compound of the present invention is administered, a dosage of 1 mg/kg is effective, whereas when the compound has an average particle size of about 50 µm (microns), a dosage of 5 mg/kg was shown to be ineffective. Similar results were obtained using the maleate salt according to the present invention. Table 1 below shows the lack of uniformity of result and linear dose dependency when the compound as known in the art having an average particle size of about 50 µm (microns) was administered to rats, as above described.

TABLE 1

| Dosages (mg/kg; per os) | Inhibition Rate | | |
|---|---|---|---|
| 0.37 | 37% | 46% | 29% |
| 1.12 | −9% | 29% | 42% |
| 3.72 | 62% | 47% | 33% |

Since 50 μm (microns) is a common particle size in pharmaceutical preparations, it was most surprising that such significant and important advantages accrued according to the present invention. Inhibition activity against stress ulcer in rats of the compound and pharmaceutically aceptable acid addition salts in the finely pulverized form of the present invention are set forth in Table 2.

TABLE 2

| | Inhibition Rate | | | | |
|---|---|---|---|---|---|
| | 0.3 | 1.0 | 3.0 | 10 | 30 mg/kg |
| Substance (I) | 33% | 45% | 63% | 70% | 85% |
| Substance (II) | 35% | 51% | 70% | 85% | — |

The data shows linear dose dependency. $ED_{50}$ values can be determined as 1.22 mg/kg (per os) and 0.90 mg/kg (per os), respectively, for substance I—2,4-diamino-6-(2,5-dichlorophenyl)-1,3,5-triazine and for substance II which is the maleate salt thereof.

It was further quite unexpected to find that when 2,4-diamino-6-(2,5-dichlorophenyl)-1,3,5-triazine having an average particle size of about 8 μm (microns) was tested for toxicity, that on intraperitoneal injection, no rats were killed with dosages up to 3000 mg/kg while the same compound having an average particle size of about 50 μm (microns) showed an $LD_{50}$ of 1740 mg/kg (1614 to 1876) when injected intraperitoneally in male rats. A similar effect was observed with the maleate salt according to the present invention. While the $LD_{50}$ of the maleate salt having an average particle size of 50 μm (microns) was determined to be 495 mg/kg (406 to 604), the maleate salt according to the present invention had an $LD_{50}$ of 835 mg/kg (696 to 1002). This decrease in toxicity is totally unexpected and represents a significant and important advance in the art.

The finely pulverized compound and pharmaceutically acceptable acid addition salts according to the present invention would appear to show decreased toxicity because the mechanism for achieving pharmacological and therapeutic activity and toxicity are quite distinct. The therapeutic results are achieved due to improved absorption, but the side effects most likely result from retention in the digestive tract.

A further advantage results from body weight increase inhibition which is one of the main side effects of the compound and salts of the present invention. When 10 mg/kg or more per day of either the compound or maleate salt is chronically administered to rats orally, body weight increase is inhibited. This is a side effect of triazines of this type. The inhibition is believed to be due to a decrease in food consumption and body weight is generally restored upon cessation of administration of the compound. However, when the compound or a pharmaceutically acceptable acid addition salt thereof according to the present invention having an average particle size of about 8 μm (microns) was administered, the appearance of the side effect was only transient and moderate and the recovery of body weight was far faster as compared to that occurring when the compound was administered having an average particle size of about 50 μm (microns). The improvement was particularly dramatic when the maleate salt according to the present invention was compared with a maleate salt having an average particle size of about 50 μm (microns), in accordance with the usual particle size for pharmaceuticals.

Figure 1 shows a change in body weights which occurs on administration of 15 mg/kg per day to male rats (10 rats in one group) over a continuous 12 day period. The average particle sizes were 8 μm (microns) for the compound according to the present invention as compared to the same compound having an average particle size of 50 μm (microns).

2,4-diamino-6-(2,5-dichlorophenyl)-1,3,5-triazine and pharmaceutically acceptable acid addition salts thereof may be finely pulverized to achieve the average particle size of 20 μm (microns) or less by using jet

Mill PJM—100NP (Nippon Newmatic MEG Co.). The pulverization is carried out by feeding 2 kg or less of the substance per hour. An average particle size of about 8 μm (microns) may be prepared in that manner. If necessary, auxiliary pulverizing agents such as starch and anhydrous silicic acid may be used. In measuring average particle size, the powder is dispersed in physiological saline solution containing one drop of Tween® 80 with the aid of an ultrasonic homogenizer for 30 seconds and measured by Coleter Counter TA—II (Coulter Electronics Co., U.S.A.) equipped with aperture tube of 100 microns.

The compound and pharmaceutically acceptable acid addition salts according to the present invention may be formulated into tablets, sugar coated or otherwise, capsules, troches, pills, granules, powders, suppositories, emulsions, suspensions and sirups, using conventional pharmaceuticaly techniques. They may be administered one or more daily as needed. Examples of auxiliary materials include:

(1) Fillers and diluents such as starch, lactose, and mannitol

(2) Binding agents such as microcrystalline cellulose, methylcellulose, other cellulose derivatives, gum arabic, gelatin, polyethylene glycol, polyvinyl alcohol, and polyvinyl pyrrolidone

(3) Wetting agents such as glycerol

(4) Disintegrating agents such as carboxylmethyl cellulose (except the sodium salts), microcrystalline cellulose, polyethyleneglycol

(5) Solubilization retarding agents such as carboxymethyl cellulose sodium salt

(6) Absorption accelerating agents such as quaternary ammonium compounds

(7) Surface active agents such as cetyl alcohol, glycerine fatty acid esters

(8) Fluidizing agents such as anhydrous silicic acid, synthetic aluminium silicate

(9) Lubricants such as talc, magnesium stearate, calcium stearate, solid polyethylene glycol

(10) Coating agents such as AEA (Trademark — Sankyo), MPM (Trademark — Tanabe), shellac, TC—5 (Trademark — Shin-Etsu).

Tablets, sugar coated tablets, capsules, trouches and pills, made from the present invention drugs may contain usual coating agents, which possess untransparent agents therein. Such materials can, for example, be manufactured from polymers or from wax.

The pharmaceutical compositions of the present invention may be formulated into a sustained release form, either by micro-incapsulation or by other techniques known per se in the pharmaceutical industry.

Examples of suitable additives to prepare suppositories are water soluble bases such as polyethylene glycol and oil bases such as cacao butter, Witepsol (Trademark — Dynamite Nobel AG). Such bases may contain surface active agents therein.

Examples of materials used for the manufacture of suspension injections, emulsions, suspensions, sirups, are as follows:

(1) Emulsification and suspensing agents such as water, ethyl alcohol, isopropyl alcohol, ethyl carbonate, benzyl alcohol, benzyl benzoate, propylene glycol, 1,3-butylene glycol, dimethylformamide, oils/fats, glycol, tetrahydrofurfuryl alcohol, polyethylene glycol

(2) Surface active agents such as sorbitan fatty acid esters, polyoxyethylene sorbitan fatty acid esters, polyoxyethylene fatty acid esters, polyoxyethylene ethers of hydrogenated castor oil, lecithin

(3) Suspension agents such as carboxymethylcellulose sodium salt, methyl cellulose, other cellulose derivatives, tragacanth, gum arabic, other natural rubbers

(4) Preservatives such as para-hydroxybenzoic acid esters, benzalkonium chloride, sorbic acid salts.

The pharmaceutical compositions according to the present invention may also contain the usual coloring agents, preservatives, perfumes, seasoning agents and sweetening agents.

The pharmaceutical compositions according to the present invention contain from about 0.1 to 99.5% and more preferably from about 0.5 to 95% of 2,4-diamino-6-(2,5-dichlorophenyl)-1,3,5-triazine or a pharmaceutically acceptable acid addition salt thereof.

In the compositions according to the present invention the compound or pharmaceutically aceptable acid addition salt thereof may be the sole therapeutic agent or the composition may contain other therapeutic agents such as digestive enzymes, antacids, inhibitants for stomach secretion, aromatic stomach agents, bitter stomach agents, protective agents for stomach mucous and anti-cholinic agents. The compositions of the present invention may also contain anti-inflammatory agents.

The route of administration is generally oral, but other routes such as rectal administration is also suitable. Generally the daily dosage will be from about 0.5 to 100 mg/kg but the precise dosage may vary according to the severity of the condition, the degree of symptoms, the past medical history of the patient and the like. When a larger amount is administered, it is generally desirable to divide the same into individual dosages.

The following non-limitative example more particularly illustrates the present invention.

## Example

2,4-Diamino-6-(2,5-dichlorophenyl)-1,3,5-triazine is powdered by supplying it at rate of not more than 2 kg/hour to a Jet Mill PJM-100 NP (Nippon Newmatic Mfg Co). The pulverized pharmaceutical is dispersed in a physiological saline solution containing one drop of Tween-80® by the use of ultrasonic homogenizer for 30 seconds and its average particle size is measured by Coulter Counter TA—II (Coleter Electronics Co, U.S.A.). The result is about 8 μm (microns).

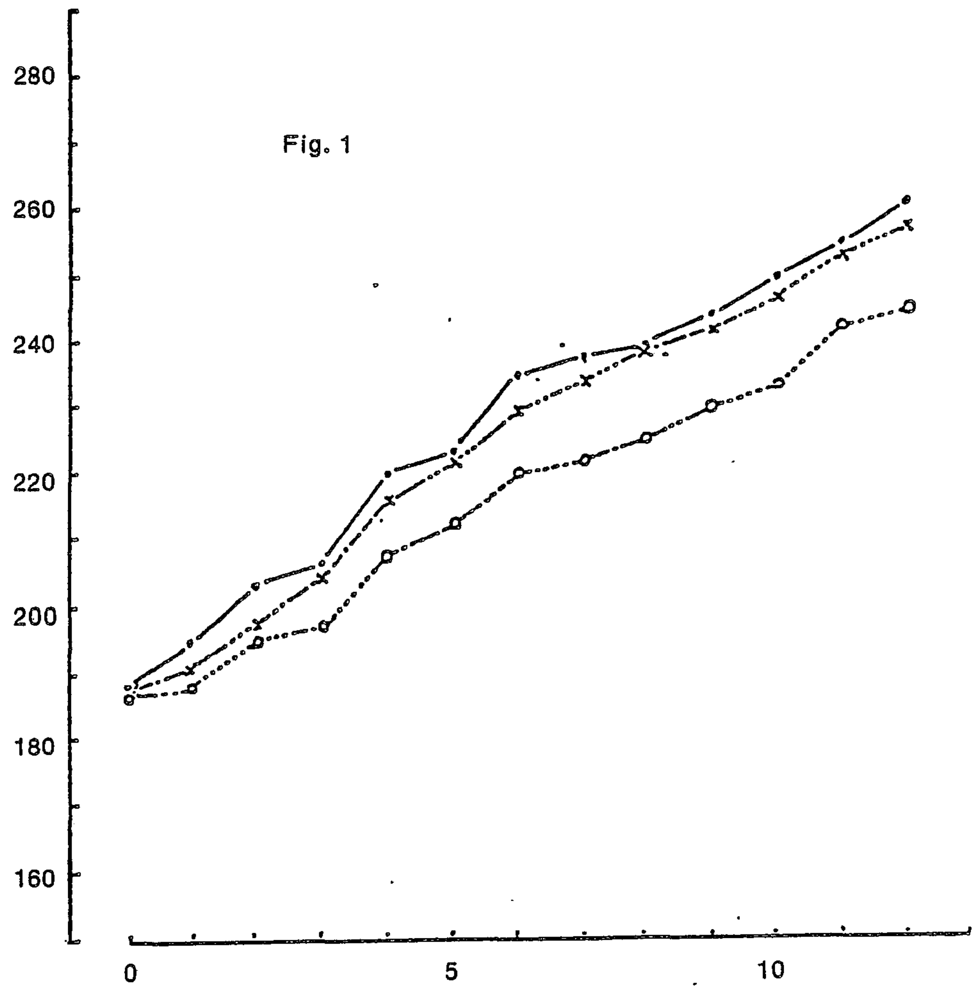


The axis of abscissa and the ordinate are administration dates and body weights, respectively. · — · means control groups, x — x means finely powdered groups according to this invention, and o — o means the ordinary conventional powder groups.

**Claims**

1. 2,4-diamino-6-(2,5-dichlorophenyl)-1,3,5-triazine or a pharmaceutically acceptable acid addition salt thereof, characterized by a finely pulverized form so that the average particle size is 20 µm (microns) or less.

2. A compound according to claim 1 in the form of the maleate salt.

3. The compound according to claim 1 or a pharmaceutically acceptable acid addition salt thereof wherein the average particle size is 5 to 10 µm (microns).

4. The maleate salt according to claim 2 where the average particle size is 5 to 10 µm (microns).

5. The compound according to claim 1 or a pharmaceutically acceptable acid addition salt thereof wherein the average particle size is about 8 µm (microns).

6. The maleate salt according to claim 2 wherein the average particle size is about 8 µm (microns).

7. 2,4-Diamino-6-(2,5-dichlorophenyl)-1,3,5-triazine according to claim 1 wherein the average particle size is about 8 µm (microns).

8. A pharmaceutical composition useful for the treatment of peptic ulcers in humans and animals which comprises a therapeutically effective amount of 2,4-diamino-6-(2,5-dichlorophenyl)-1,3,5-triazine or a pharmaceutically acceptable acid addition salt thereof, characterized by a finely pulverized form so that the average particle size is 20 µm (microns) or less, in combination with a pharmaceutically acceptable carrier.

9. A composition according to claim 8 wherein the compound is in the form of the maleate salt.

10. A composition according to claim 8 wherein the average particle size is 5 to 10 µm (microns).

11. A composition according to claim 9 wherein the average particle size is 5 to 10 µm (microns).

12. A composition according to claim 8 wherein the average particle size is about 8 µm (microns).

13. A composition according to claim 9 wherein the average particle size is about 8 µm (microns).

14. A composition according to claim 8 wherein the compound is 2,4-diamino-6-(2,5-dichlorophenyl)-1,3,5-triazin and the average particle size is about 8 µm (microns).

15. A composition according to claim 8 in oral administration form.

16. A composition according to claim 8 in rectal administration form.

**Patentansprüche**

1. 2,4-Diamino-6-(2,5-dichlorphenyl)-1,3,5-triazin oder seine pharmazeutisch akzeptierbaren Säureadditionssalze, gekennzeichnet durch eine fein pulverisierte Form, so daß die durchschnittliche Teilchengröße 20 µm (Mikron) oder weniger beträgt.

2. Verbindung nach Anspruch 1 in Form des Maleat-Salzes.

3. Verbindung nach Anspruch 1 oder eines ihrer pharmazeutisch akzeptablen Säureadditionssalze, wobei die durchschnittliche Teilchengröße 5 bis 10 µm (Mikron) beträgt.

4. Maleatsalz nach Anspruch 2, wobei die durchschnittliche Teilchengröße 5 bis 10 µm (Mikron) beträgt.

5. Verbindung nach Anspruch 1 oder eines ihrer pharmazeutisch akzeptablen Säureadditionssalze, wobei die durchschnittliche Teilchengröße etwa 8 µm (Mikron) beträgt.

6. Maleatsalz nach Anspruch 2, wobei die durchschnittliche Teilchengröße etwa 8 µm (Mikron) beträgt.

7. 2,4-Diamino-6-(2,5-dichlorphenyl)-1,3,5-triazin nach Anspruch 1, wobei die durchschnittliche Teilchengröße etwa 8 µm (Mikron) beträgt.

8. Pharmazeutische Zusammensetzung für die Behandlung von Magengeschwüren bei Menschen und Tieren, bestehend aus einer therapeutisch wirksamen Menge 2,4-Diamino-6-(2,5-dichlorphenyl)-1,3,5-triazin oder einem seiner pharmazeutisch akzeptablen Säureadditionssalze, gekennzeichnet durch eine fein pulverisierte Form, so daß die durchschnittliche Teilchengröße 20 µm (Mikron) oder weniger beträgt, in Kombination mit einem pharmazeutisch akzeptablen Träger.

9. Zusammensetzung nach Anspruch 8, wobei die Verbindung in Form des Maleatsalzes vorliegt.

10. Zusammensetzung nach Anspruch 8, wobei die durchschnittliche Teilchengröße 5 bis 10 µm (Mikron) beträgt.

11. Zusammensetzung nach Anspruch 9, wobei die durchschnittliche Teilchengröße 5 bis 10 µm (Mikron) beträgt.

12. Zusammensetzung nach Anspruch 8, wobei die durchschnittliche Teilchengröße etwa 8 µm (Mikron) beträgt.

13. Zusammensetzung nach Anspruch 9, wobei die durchschnittliche Teilchengröße etwa 8 µm (Mikron) beträgt.

14. Zusammensetzung nach Anspruch 8, wobei die Verbindung 2,4-Diamino-6-(2,5-dichlorphenyl)-1,3,5-triazin ist und die durchschnittliche Teilchengröße etwa 8 µm (Mikron) beträgt.

15. Zusammensetzung nach Anspruch 8, in oral verabreichbarer Form.

16. Zusammensetzung nach Anspruch 8, in rektal verabreichbarer Form.

**Revendications**

1. 2,4-diamino-6-(2,5-dichlorophényl)-1,3,5-triazine ou un de ses sels d'addition d'acide pharmaceutiquement acceptables, caractérisé par une forme finement pulvérisée telle que la dimension moyenne des particules soit de 20 µm (microns) ou moins.

2. Composé selon la revendication 1 sous la forme de maléate.

3. Composé selon la revendication 1 ou un de ses sels d'addition d'acide pharmaceutiquement acceptables, caractérisé en ce que la dimension moyenne des particules est de 5 à 10 µm (microns).

4. Maléate selon la revendication 2, caractérisée en ce que la dimension moyenne des particules est de 5 à 10 µm (microns).

5. Composé selon la revendication 1 ou un de ses sels d'addition d'acides pharmaceutiquement acceptables, caractérisé en ce que la dimension moyenne des particules est d'environ 8 µm (microns).

6. Maléate selon la revendication 2, caractérisée en ce que la dimension moyenne des particules est d'environ 8 µm (microns).

7. 2,4-diamino-6-(2,5-dichlorophényl)-1,3,5-triazine selon la revendication 1, caractérisé en ce que la dimension moyenne des particules est d'environ 8 µm (microns).

8. Composition pharmaceutique utile pour le traitement d'ulcères peptiques chez l'homme et chez les animaux, comprenant une quantité thérapeutiquement efficace de 2,4-diamino-6-(2,5-dichlorophényl)-1,3,5-triazine ou d'un de ses sels d'addition d'acide pharmaceutiquement acceptables, caractérisée par une forme finement pulvérisée telle que la dimension moyenne des particules est de 20 µm (microns) ou moins, en combinaison avec un véhicule pharmaceutiquement acceptable.

9. Composition selon la revendication 8, caractérisée en ce que le composé est sous la forme de maléate.

10. Composition selon la revendication 8, caractérisée en ce que la dimension moyenne des particules est de 5 à 10 µm (microns).

11. Composition selon la revendication 9, caractérisée en ce que la dimension moyenne des particules est de 5 à 10 µm (microns).

12. Composition selon la revendication 8, caractérisée en ce que la dimension moyenne des particules est d'environ 8 μm (microns).

13. Composition selon la revendication 9, caractérisée en ce que la dimension moyenne des particules est d'environ 8 μm (microns).

14. Composition selon la revendication 8, caractérisée en ce que le composé est la 2,4-diamino-6-(2,5-dichlorophényl)-1,3,5-triazine et que la dimension moyenne des particules est d'environ 8 μm (microns).

15. Composition selon la revendication 8, sous une forme adaptée à l'administration par voie orale.

16. Composition selon la revendication 8, sous une forme adaptée à l'administration par voie rectale.